(19) Europäisches Patentamt / European Patent Office / Office européen des brevets

(11) **EP 3 407 068 A1**

(12) **EUROPEAN PATENT APPLICATION**

(43) Date of publication:
**28.11.2018 Bulletin 2018/48**

(51) Int Cl.:
***G01N 33/68*** (2006.01)

(21) Application number: **17172735.7**

(22) Date of filing: **24.05.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
- **CHATTERJEA, Supriyo**
  **5656 AE Eindhoven (NL)**
- **DE JAGER, Marinus Karel Johannes**
  **5656 AE Eindhoven (NL)**

- **RMAILE, Amir Hussein**
  **5656 AE Eindhoven (NL)**
- **KOOIJMAN, Gerben**
  **5656 AE Eindhoven (NL)**
- **VAN HARTSKAMP, Michael Alex**
  **5656 AE Eindhoven (NL)**
- **PRESHAW, Philips**
  **5656 AE Eindhoven (NL)**
- **TAYLOR, John**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **DIAGNOSTICS OF MILD OR ADVANCED PERIODONTITIS BASED ON SALIVARY IL-1BETA AND MMP-9**

(57) Disclosed is an *in vitro* method for assessing whether a human patient suffering from periodontitis has mild periodontitis or advanced periodontitis. The method is based on the insight to determine a selection of three biomarker proteins. Accordingly, in a sample of saliva a patient suffering from periodontitis, the concentrations are measured of the proteins Interleukin-1β (IL1β) Matrix metalloproteinase 9 (MMP-9) and at least one of the proteins: Interleukin-6 (IL6), and Matrix metalloproteinase 3 (MMP-3). Based on the concentrations as measured, a value is determined reflecting the joint concentrations for said proteins. This value is compared with a threshold value reflecting in the same manner the joint concentrations associated with advanced periodontitis. The comparison allows assessing whether the testing value is indicative of the presence of advanced periodontitis or of mild periodontitis in said patient. Thereby, typically, a testing value reflecting a joint concentration below the joint concentration reflected by the threshold value is indicative for mild periodontitis in said patient, and a testing value reflecting a joint concentration at or above the joint concentration reflected by the threshold value, is indicative for advanced periodontitis in said patient.

Fig. 1

EP 3 407 068 A1

**Description**

FIELD OF THE INVENTION

The invention is in the field of oral care, and pertains to saliva-based diagnostics of periodontal disease. Particularly, the invention pertains to a kit and method for distinguishing mild from advanced periodontitis.

BACKGROUND OF THE INVENTION

Periodontitis is a chronic multifactorial inflammatory disease caused by oral microorganisms and characterized by progressive destruction of the hard (bone) and soft (periodontal ligament) tissues, ultimately leading to tooth mobility and loss. This is to be distinguished from gingivitis which is a reversible infection and inflammation of the gum tissues. Inflammatory periodontitis is one of the most prevalent chronic human diseases and a major cause of adult tooth loss. In addition to the substantial negative impact of periodontitis on oral health, there is also mounting evidence that periodontitis has systemic consequences and that it is a risk factor for several systemic diseases, including heart diseases (e.g. atherosclerosis, stroke), diabetes, pregnancy complications, rheumatoid arthritis and respiratory infections.
Early and accurate diagnosis of periodontal disease, thus, is important from both an oral and overall health perspective.
Periodontal diseases are still poorly diagnosed in general dental practice (Morgan RG (2001). Brit Dent J 191: 436-41), resulting in relatively low rates of therapeutic intervention and significant amounts of untreated cases. Current diagnosis relies on imprecise, subjective clinical examination of oral tissue condition (color, swelling, extent of bleeding on probing, probing pocket depth; and bone loss from oral x-rays) by dental professionals. These conventional methods are time consuming, and some of the techniques used (pocket-depth, x-ray) reflect historic events, such as past disease activity, rather than current disease activity or susceptibility to further disease. Hence, more objective, faster, accurate, easier-to-use diagnostics which preferably may also be performed by non-specialists are desirable. Thereby it is desirable to measure current disease activity, and possibly a subject's susceptibility to further periodontal disease.
Saliva or oral fluids have long been advocated as a diagnostic fluid for oral and general diseases, and with the advent of miniaturized biosensors, also referred to as labon-a-chip, point of care diagnostics for rapid chair-side testing have gained greater scientific and clinical interest Especially for periodontal disease detection, inflammatory biomarkers associated with tissue inflammation and breakdown may easily end up in saliva due to proximity, suggesting saliva has strong potential for periodontal disease detection. Indeed, this area thus has gained significant interest and encouraging results have been presented, yet no definite test has emerged yet.
Biomarkers represent biological indicators that underpin clinical manifestations, and as such are objective measures by which to diagnose clinical outcomes of periodontal disease. Ultimately, proven biomarkers could be utilized to assess risk for future disease, to identify disease at the very earliest stages, to identify response to initial therapy, and to allow implementation of preventive strategies.
Previous limitations to the development of point-of-care tests for salivary biomarkers included a lack of technologies that were adaptable to chair-side applications and an inability to analyze multiple biomarkers in individual samples. Also the selection of which multiple biomarkers to include in such a test has not been adequately addressed in the literature nor implemented in practical tests.
Moreover, periodontitis can manifest itself across the entire spectrum of severity ranging from mild to advanced forms of the disease. In order to easily assess the severity of the condition, dentists often classify patients suffering from periodontitis into two groups - those suffering from *mild* periodontitis, and those suffering from *advanced* periodontitis. The available methods of making such an assessment, however, involve a labor intensive process that a dentist will not perform routinely on every patient and/or on every visit, and that is impossible to perform by a consumer (self-diagnosis).
It would be desired to provide a simpler process, and particularly a process that requires only that a small saliva sample is taken from a patient, and possibly by the patient him- or herself. It is desired that such a sample be entered into an *in vitro* diagnostic device, which will allow, based on measurement, a classification of the saliva sample such that it can return an indication of the likelihood that the patient is to be classified as suffering from mild periodontitis or as suffering from advanced periodontitis.

SUMMARY OF THE INVENTION

In order to better address the foregoing desires, the invention, in one aspect, concerns an *in vitro* method for assessing whether a human patient, known to have periodontitis, has mild periodontitis or advanced periodontitis, the method comprising detecting, in a sample of saliva from said patient, the concentrations of the proteins: Interleukin-1$\beta$ (IL1$\beta$), Matrix metalloproteinase 9 (MMP-9), and at least one of the proteins Interleukin-6 (IL6) Matrix metalloproteinase 3 (MMP-3); determining a testing value reflecting the joint concentrations determined for said proteins; comparing the testing value with a threshold value reflecting in the same manner the joint concentrations associated with advanced periodontitis, so as to assess whether the testing value is indicative for mild periodontitis or for advanced periodontitis in said patient.

In another aspect, the invention presents the use of the proteins Interleukin-1β (IL1β), Matrix metalloproteinase 9 (MMP-9), and at least one of the proteins Interleukin-6 (IL6) Matrix metalloproteinase 3 (MMP-3), as biomarkers in a saliva sample of a human patient suffering from periodontitis, for assessing whether the patient has mild periodontitis or advanced periodontitis.

In a further aspect, the invention resides in a system for classifying the type of periodontitis in a human patient suffering from said periodontitis, the system comprising:

- a container for receiving an oral sample of a subject therein, the container provided with detection means which are able and adapted to detect in the oral sample an expression of a set of biomarkers, the set comprising Interleukin-1β (IL1β), Matrix metalloproteinase 9 (MMP-9), and at least one of the proteins Interleukin-6 (IL6) Matrix metalloproteinase 3 (MMP-3);
- a data connection to an interface, particularly a graphical user interface, capable of presenting information, preferably also capable of putting in information, said interface being either a part of the system or a remote interface;
- a processor able and adapted to determine from the detected expression of the set of biomarkers in an oral sample of the patient a diagnosis result comprising an indication of the patient having mild periodontitis or advanced periodontitis.

Optionally one or more of the foregoing items, particularly the processor, are enabled to function "in the cloud", i.e., not on a fixed machine, but by means of an internet-based application.

In a still further aspect, the invention provides a kit for determining the concentration of at least three biomarkers for periodontitis in saliva, said kit comprising three binding agents, each binding agent directed to a different biomarker, wherein a first binding agent is capable of binding IL1β, a second binding agent is capable of binding MMP-9, and a third binding agent is capable of binding one of IL6 and MMP-3.

In yet another aspect, the invention provides an *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins: IL1β, MMP-9, and at least one of IL6 and MMP-3, and comparing the concentrations, whereby a difference in any one, two or all three of the concentrations, reflects a change in status.

BRIEF DESCRIPTION OF THE DRAWINGS

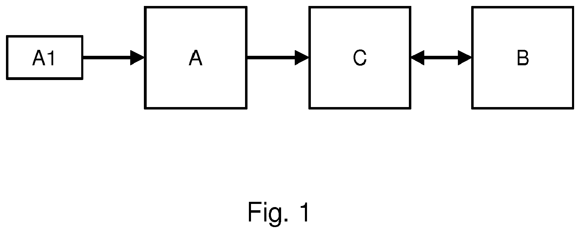

Fig. 1 schematically represents a system for use in the method as described in this disclosure.

DETAILED DESCRIPTION OF THE INVENTION

In a general sense, the invention is based on the judicious insight that as few as three proteins can serve as biomarkers in a sample of saliva of a human patient suffering from periodontitis, for classifying said periodontitis as being in either of two categories, one category being advanced periodontitis, the other category being mild or moderate (i.e., not advanced, hereinbefore and hereinafter the term "mild periodontitis" will include moderate periodontitis, unless indicated otherwise). The latter category is hereinafter collectively indicated as mild periodontitis.

These proteins are Interleukin-1β (IL1β), Matrix metalloproteinase 9 (MMP-9), and either or both of Interleukin-6 (IL6) and Matrix metalloproteinase 3 (MMP-3).

IL-1β is a member of the interleukin 1 family of cytokines. This cytokine is produced by activated macrophages as a proprotein, which is proteolytically processed to its active form by caspase 1 (CASP1/ICE). This cytokine is an important mediator of the inflammatory response, and is involved in a variety of cellular activities, including cell proliferation, differentiation, and apoptosis.

IL-6 is an interleukin that acts as both a pro-inflammatory cytokine and an anti-inflammatory myokine. In humans, it is encoded by the IL6 gene.

Interleukin 6 is secreted by T cells and macrophages to stimulate immune response, e.g. during infection and after trauma, especially burns or other tissue damage leading to inflammation. IL-6 also plays a role in fighting infection, as IL-6 has been shown in mice to be required for resistance against bacterium Streptococcus pneumoniae. In addition, osteoblasts secrete IL-6 to stimulate osteoclast formation. Smooth muscle cells in the tunica media of many blood vessels also produce IL-6 as a pro-inflammatory cytokine.

MMPs are a family of enzymes that are responsible for the degradation of extracellular matrix components such as collagen, proteoglycans, laminin, elastin, and fibronectin. They play a central role in the periodontal ligament (PDL) remodelling, both in physiological and pathological conditions. The MMP-3 enzyme degrades collagen types II, III, IV, IX, and X, proteoglycans, fibronectin, laminin, and elastin. MMP-9, also known as 92 kDa type IV collagenase, 92 kDa gelatinase or gelatinase B (GELB), is a matrixin, a class of enzymes that belong to the zinc-metalloproteinases family involved in the degradation of the extracellular matrix.

The four proteins mentioned above are known in the art. The skilled person is aware of their structure, and of methods to detect them in an aqueous sample, such as a saliva sample. Hereinafter the aforementioned protein biomarkers are collectively referred to as "the biomarker panels of the invention." A biomarker panel of the invention, in one embodiment, consist of the four protein biomarkers identified in the invention, i.e., IL1β, IL6, MMP-3, and MMP-9. Preferably, a biomarker panel of the invention consists of not more than three of the protein biomarkers identified in the invention, i.e., IL1β, IL6, and MMP-9, or, IL1β, MMP-3, and MMP-9. In addition to the biomarker panels of the invention, other biomarkers and or data, such as demographic data (e.g., age, sex) can be included in a set of data applied for the determination of the type of periodontitis. Preferred extended biomarker panels are:

IL1B + MMP-9 + IL6 + MMP-3
IL1B + MMP-9 + IL6 + MMP-8
IL1B + MMP-9 + IL6 + MMP-3 + MMP-8
IL1B + MMP-9 + IL6 + MMP-8 + HGF (Hepatocyte growth factor).

However, a desirable advantage of the present invention is that the classification of periodontitis in a patient can be determined by measuring preferably not more than four biomarkers, and more preferably measuring only three biomarkers, with the biomarker panel IL1β, IL6, and MMP-9 being preferred. Particularly, the determination does not need to involve the use of other data, which advantageously provides a simple and straightforward diagnostic test.

The method, as desired, requires only that a small saliva sample, e.g. a dropsize, is taken from the subject. The size of the sample will typically range of from 0.1 μl to 2 ml, such as 1-2 ml, whereby smaller amounts, e.g., 0. 1 to 100 μl can be used for *in vitro* device processing, and whereby taking a larger sample, such as up to 20 ml, such as 7.5 to 17 ml, is also possible.

This sample is entered into an *in vitro* diagnostic device, which measures the concentrations of the at least three proteins involved, and which returns a diagnostic outcome, classifying the subject on the basis of a likelihood of having mild periodontitis or advanced periodontitis.

The ease of use of this invention will make it possible to test the majority of dental patients with periodontitis, or with a high risk for developing periodontitis, on a regular basis (e.g. as part of a regular dental check or even at home). This allows, *inter alia,* detecting the presence of mild periodontitis before it proceeds to advanced periodontitis, and thus enables more timely taking oral care measures to prevent periodontitis from advancing. Or, e.g., with patients known to be at high risk for periodontitis, and tested for the first time, the method allows to identify whether the periodontitis is mild or advanced. Also, the method can be applied after treatment of a patient previously diagnosed with advanced periodontitis, in order to check whether the periodontitis has improved so as to become mild. Particularly, the method is also suitable for self-diagnosis, whereby the steps of taking the sample and entering it into a device can be conducted by the patient him- or herself.

A method of the invention typically comprises detecting the aforementioned at least three proteins making up a biomarker panel of the invention, and optional further biomarker proteins, by using one or more detection reagents. By "detecting" is meant measuring, quantifying, scoring, or assaying the concentration of the biomarker proteins. Methods of evaluating biological compounds, including biomarker proteins, are known in the art. It is recognized that methods of detecting a protein biomarker include direct measurements and indirect measurements. One skilled in the art will be able to select an appropriate method of assaying a particular biomarker protein.

A "detection reagent" is an agent or compound that specifically (or selectively) binds to, interacts with or detects the protein biomarker of interest. Such detection reagents may include, but are not limited to, an antibody, polyclonal antibody, or monoclonal antibody that preferentially binds the protein biomarker.

The phrase "specifically (or selectively) binds" or "specifically (or selectively) immunoreactive with," when referring to a detection reagent, refers to a binding reaction that is determinative of the presence of the protein biomarker in a heterogeneous population of proteins and other biologics. Thus, under designated immunoassay conditions, the specified detection reagent (e.g. antibody) binds to a particular protein at least two times the background and does not substantially bind in a significant amount to other proteins present in the sample. Specific binding under such conditions may require an antibody that is selected for its specificity for a particular protein. A variety of immunoassay formats may be used to select antibodies specifically immunoreactive with a particular protein. For example, solid-phase ELISA immunoassays (enzyme linked immunosorbent assay) are routinely used to select antibodies specifically immunoreactive with a protein (*see, e.g.,* Harlow & Lane, Antibodies, A Laboratory Manual (1988), for a description of immunoassay formats and conditions that can be used to determine specific immunoreactivity). Typically a specific or selective reaction will be at least twice the background signal or noise and more typically more than 10 to 100 times the background.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region.

Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive.

The biomarker protein(s) of the invention can be detected in a sample by any means. Preferred methods for biomarker detection are antibody-based assays, protein array assays, mass spectrometry (MS) based assays, and (near) infrared spectroscopy based assays. For example, immunoassays, include but are not limited to competitive and non-competitive assay systems using techniques such as Western blots, radioimmunoassays, ELISA, "sandwich" immunoassays, immunoprecipitation assays, precipitin reactions, gel diffusion precipitin reactions, immunodiffusion assays, fluorescent immunoassays and the like. Such assays are routine and well known in the art. Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1 % sodium deoxycholate, 0.1% SDS, 0.15 M NaCl, 0.01 M sodium phosphate at pH 7.2, 1 % Trasylol) supplemented with protein phosphatase and/or protease inhibitors (e.g., EDTA, PMSF, aprotinin, sodium vanadate), adding an antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with Sepharose beads).

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that can be modified to increase the signal detected and to reduce the background noise.

ELISAs typically comprise preparing antigen (i.e. the biomarker protein of interest or fragment thereof), coating the well of a 96- well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

Since multiple markers are used, a threshold is determined on the basis of the joint concentrations of these biomarkers. This threshold determines whether a patient is classified as having mild periodontitis or advanced periodontitis. The invention reflects the insight that periodontitis can be detected, as being mild or advanced, with sufficient accuracy based on a measurement of the combination of biomarkers as indicated above.

This insight supports another aspect, the invention, which is the use of the proteins IL1β, MMP-9, and at least one of IL6 and MMP-3, as biomarkers in a saliva sample of a human patient suffering from periodontitis, for assessing whether the patient has mild periodontitis or advanced periodontitis.

This use can be implemented in a method as substantially described hereinbefore and hereinafter.

The method of the invention comprises determining a testing value reflecting the joint concentrations measured for said proteins. A joint concentration value can be any value obtained by input of the concentrations as determined and an arithmetic operation of these values. This can, e.g., be a simple addition of the concentrations. It can also involve multiplying each concentration with a factor reflecting a desired weight of these concentrations, and then adding up the results. It can also involve multiplying the concentrations with each other, or any combination of multiplication, division, subtraction, exponentiation, and addition. It can further involve raising concentrations to some power.

The resulting joint concentration value is compared with a threshold value reflecting in the same manner the joint concentrations associated with the presence of advanced periodontitis. The comparison allows assessing whether the testing value is indicative of the presence of advanced periodontitis in the patients whose saliva is subjected to the test, or of mild periodontitis.

The threshold value can, e.g., be the joint concentration value, obtained in the same manner on the basis of the concentrations determined for the same proteins in a reference sample associated with the presence of advanced periodontitis, i.e. in a patient diagnosed with advanced periodontitis. Typically, thereby a value reflecting the same or higher joint concentration is indicative of the presence of advanced periodontitis in a tested patient. Analogously, a value reflecting a lower joint concentration in the saliva of a tested periodontitis patient, indicates that the periodontitis is mild or moderate (i.e., not advanced). However, it will be understood that it is also possible to calculate a threshold value (e.g. by using a negative multiplier) such that a testing value indicating advanced periodontitis would be below the threshold, and a testing value indicating mild periodontitis, would be above the threshold.

The threshold value can also be determined on the basis of measuring the concentrations of the present biomarker proteins in a set of samples, including patients with a known diagnosis of advanced periodontitis and not advanced (mild and/or moderate) periodontitis. Thereby the measured concentration values can be subjected to statistical analysis, possibly including machine learning methods, allowing to discriminate, with the desired sensitivity and specificity, patients classified as having mild or moderate periodontitis and patients classified as patients suffering from advanced periodontitis. Therefrom, the desired threshold value can be obtained. On the basis of this threshold value, a sample to be tested can be subjected to the same concentration measurement, and the concentration values are then processed, in the same manner in which the threshold value is obtained, so as to determine a joint concentration value that can be compared with the threshold, thus allowing the tested sample to be classified as having mild or advanced periodontitis.

In an interesting embodiment, the joint concentration value is obtained in the form of a score as follows. A numerical value (protein concentration values in e.g. ng/ml) is assigned to each measurement, and these values are used in a linear or non-linear combination to calculate a score between zero and one. In the event that the threshold value is determined on the basis of a set of subjects as mentioned above, the score between 0 and 1 is typically calculated with the sigmoid function that takes the joint concentration as input (as shown further on).

When the score exceeds a certain threshold, the method indicates that the patient has advanced periodontitis. The threshold may be chosen based on the desired sensitivity and specificity.

It will be understood that in performing a 'mild or advanced periodontitis classification' on a subject, in accordance with the invention, this is on subjects that can be assumed to suffer from periodontitis. This can either be known from e.g. a previously performed diagnosis of periodontitis, though perhaps without ability to differentiate the extent of it, or, e.g., assumed from the subject's oral health condition record.

Clinical definitions as acknowledged in the art are based on the following:

*Gingival Index (GI)*

A full mouth gingival index will be recorded based on the Lobene Modified Gingival Index (MGI) rated on a scale of 0 to 4, where:

- 0 = absence of inflammation,
- 1 = mild inflammation; slight change in color little change in texture of any portion of but not the entire margin or papillary gingival unit,
- 2 = mild inflammation; but involving entire margin or papillary unit,
- 3 = moderate inflammation; glazing, redness, oedema and/or hypertrophy of margin or papillary unit,
- 4 = severe inflammation; marked redness, oedema and/or hypertrophy of marginal or papillary gingival unit, spontaneous bleeding, congestion, or ulceration].

*Probing depths (PD)*

Probing depths will be recorded to the nearest mm using a manual UNC-15 periodontal probe. Probing depth is the distance from the probe tip (assumed to be at the base of the pocket) to the free gingival margin.

*Gingival recession (REC)*

Gingival recession will be recorded to the nearest mm using a manual UNC-15 periodontal probe. Gingival recession is the distance from the free gingival margin to the cemento-enamel junction. Gingival recession will be indicated as a positive number and gingival overgrowth will be indicated as a negative number.

*Clinical attachment loss (CAL)*

Clinical attachment loss will be calculated as the sum of probing depth + recession at each site.

*Bleeding on probing (BOP)*

Following probing, each site will be assessed for bleeding on probing, if bleeding occurs within 30s of probing, a score of 1 will be assigned for the site, otherwise a score of 0 will be assigned.

The resulting subject group (patient group) definition is as follows, whereby the mild-moderate periodontitis group and the advanced periodontitis group are relevant to the present invention:

- Healthy group (H): PD $\leq$ 3 mm in all sites (but would allow up to four 4 mm pockets at distal of last standing molars), no sites with interproximal attachment loss, GI of $\geq$ 2.0 in $\leq$ 10% sites, %BOP scores $\leq$ 10%;
- Gingivitis group (G): GI $\geq$ 3.0 in > 30% of sites, no sites with interproximal attachment loss, no sites with PD > 4 mm , %BOP scores > 10%;
- Mild-moderate periodontitis group (MP): interproximal PD of 5-7 mm, (equating to approximately 2-4 mm CAL) at $\geq$ 8 teeth, %BOP scores > 30%;
- Advanced periodontitis group (AP): interproximal PD of $\geq$ 7 mm, (equating to approximately $\geq$ 5 mm CAL) at $\geq$ 12 teeth, %BOP scores > 30%.

In an embodiment, the method of the invention makes use of a system as represented schematically in Fig. 1. The system can be a single apparatus having various device components (units) integrated therein. The system can also have its various components, or some of these components, as separate apparatuses. The components shown in Fig. 1 are a measurement device (A), a graphical user interface (B) and a computer processing unit (C).

As mentioned above, the system of the invention comprises a data connection to an interface, whereby the interface itself can be a part of the system or can be a remote interface. The latter refers to the possibility to use a different apparatus, preferably a handheld apparatus such as a smartphone or a tablet computer, for providing the actual interface. The data connection in such cases will preferably involve wireless data transfer such as by Wi-Fi or Bluetooth, or by other techniques or standards.

The measurement device (A) is configured to receive a saliva sample, for example by putting a drop of saliva on a cartridge (A1), which can be inserted into the device (A). The device can be an existing device that is capable to determine, from the same saliva sample, the concentrations of at least the proteins IL1$\beta$, MMP-9, and at least one of the proteins IL6 and MMP-3.

The measurement device (A) should be able to receive a saliva sample, for example by putting a drop of saliva on a cartridge (A1), which can be inserted into the device (A). The device may be an existing device that is capable to determine, from the same saliva sample, the concentrations of at least the proteins IL1-beta and MMP-9, and optionally at least one of the proteins IL6 and MMP-3.

The processing unit (C) receives numerical values for the protein concentrations from part (A). The unit (C) is provided with software (typically embedded software) allowing it to calculate a score (S) between 0 and 1. The software further includes a numerical value for the threshold (T). If the calculated value (S) exceeds (T), unit (C) will output an indication (I) of 'advanced periodontitis' to the GUI (B), otherwise it will output 'mild periodontitis'. A further embodiment may use the specific value of (S) to indicate the certainty with which the indication (I) is made. This can be a probability score, whereby 0.5 is a possible threshold value, and e.g. a score S=0.8 would indicate the probability of advanced periodontitis. Interesting options are:

Based on the score S, one can directly indicate a certainty, i.e. S=0.8 means 80% certainty of advanced periodontitis;

- Based on the score S one can make a binary or tertiary indication:
- S<T -> mild periodontitis, S$\geq$T -> advanced periodontitis;
- S<R1 -> mild periodontitis, R1$\leq$S<R2 -> inconclusive,

- S≥R2 -> advanced periodontitis;

In addition, it is possible to attach a certainty to such a binary or tertiary indication. This certainty will be determined by the distance of the score S from the chosen threshold(s) (T,R1,R2).

A specific calculation of the score can be implemented, e.g., by means of a sigmoid function applying the following formula:

$$S = \frac{1}{1 + \exp(-(c_0 + \sum_{i=1}^{N} c_i B_i))}$$

Therein $N$ is the number of proteins/biomarkers used. $c_0$, $c_1$, etc. are coefficients (numerical values) and $B_1, B_2$, etc. are the respective protein concentrations.

Determining of the coefficients can be done by a training procedure:

- Select N1 subjects with advanced periodontitis and N2 subjects with mild periodontitis.

The subjects without mild periodontitis are considered to have score S=0, the subjects with advanced periodontitis are considered to have score S=1.

- Take a saliva sample from each subject and determine the protein concentrations of a combination of biomarkers as explained above.
- Perform logistic regression between the protein concentrations and the scores.

    * Other regression or machine learning methods (linear regression, neural network, support vector machine) may be used to train a classifier that predicts whether a subject has gingivitis or a healthy oral condition based on the protein concentrations.

With reference to the aforementioned system, the invention also provides, in a further aspect, a system for classifying the type of periodontitis in a human patient suffering from said periodontitis, the system comprising:

- a container for receiving an oral sample of a subject therein, the container provided with detection means which are able and adapted to detect in the oral sample an expression of a set of biomarkers, the set comprising IL1β, IL6, and at least one of MMP-9 and MMP-8; As explained above, such means are known, and easily accessible to the skilled person;
- an interface (or a data connection to remote interface), particularly a graphical user interface (GUI), capable of presenting information; a GUI is a type of user interface that allows users to interact with electronic devices through graphical icons and visual indicators such as secondary notation, instead of text-based user interfaces, typed command labels or text navigation (none of such interface types being excluded in the present invention); GUIs are generally known, and are used typically in handheld mobile devices such as MP3 players, portable media players, gaming devices, smartphones and smaller household, office and industrial controls; as said, the interface optionally can also be chosen so as to be capable of putting in information,such as, e.g., the age of the subject, sex, BMI (Body Mass Index);
- a processor able and adapted to determine from the detected expression of the set of biomarkers in an oral sample of the patient a diagnosis result comprising an indication of the patient having mild periodontitis or advanced periodontitis.

The invention also provides, either separately or as part of the aforementioned system, a kit for determining the concentration of at least three biomarkers for periodontitis in saliva, said kit comprising three binding agents, each binding agent directed to a different biomarker, wherein a first binding agent is capable of binding IL1β, a second binding agent is capable of binding MMP-8, and a third binding agent is capable of binding one of IL6 and MMP-3. As discussed above with reference to the method of the invention, the kit may comprise more binding agents, such as particularly for the other of IL6 and MMP-3, and/or for other proteins. In a preferred embodiment the binding agents made available in the kit consist of the binding agents for the selection of three proteins making up a 3-biomarker panel of the invention, as mentioned.

Preferably said kits comprise a solid support, such as a chip, a microtiter plate or a bead or resin comprising said binding agents. In some embodiments, the kits comprise mass spectrometry probes, such as ProteinChip™.

The kits may also provide washing solutions and/or detection reagents specific for either unbound binding agent or for said biomarkers (sandwich type assay).

In an interesting aspect, the recognition of a biomarker panel of the invention is applied in monitoring the status of periodontitis in a human patient, over time. Accordingly, the invention also provides an *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins: IL1β, MMP-8, and at least one of IL6 and MMP-3, and comparing the concentrations, whereby a difference of preferably at least two, and more preferably all three concentrations, reflects a change in status. This difference can be reviewed as a difference in concentrations, thus allowing a direct comparison without first generating a number between 0 and 1, or any other classification. It will be understood that the measurements received at both points in time can also be processed in just the same manner as done when determining the mild or advanced periodontitis as above.

The invention will be further illustrated with reference to the following nonlimiting example.

Example

In a clinical study with 60 subjects, 32 of whom were diagnosed with mild periodontitis (including moderate periodontitis) and 28 with advanced periodontitis, ROC (Receiver-Operator-Characteristic) Area-Under-the Curve (AUC) values were obtained.

In statistics, a receiver operating characteristic curve, or ROC curve, is a graphical plot that illustrates the performance of a binary classifier system as its discrimination threshold is varied. The curve is created by plotting the true positive rate (TPR) against the false positive rate (FPR) at various threshold settings. The true-positive rate is also known as sensitivity, recall or *probability of detection* in machine learning. The false-positive rate is also known as the fall-out or *probability of false alarm* and can be calculated as (1 - specificity). The ROC curve is thus the sensitivity as a function of fall-out. In general, if the probability distributions for both detection and false alarm are known, the ROC curve can be generated by plotting the cumulative distribution function (area under the probability distribution from - ∞ to the discrimination threshold) of the detection probability on the y-axis, versus the cumulative distribution function of the false-alarm probability on the x-axis. The accuracy of the test depends on how well the test separates the group being tested into those with and without the disease in question. Accuracy is measured by the area under the ROC curve. An area of 1 represents a perfect test; an area of 0.5 represents a worthless test. A guide for classifying the accuracy of a diagnostic test is the traditional academic point system:

- 0.90-1 = excellent (A)
- 0.80-0.90 = good (B)
- 0.70-0.80 = fair (C)
- 0.60-0.70 = poor (D)
- 0.50-0.60 = fail (F)

Based on the foregoing, in the results of the aforementioned clinical study, an ROC AUC value of above 0.75 is considered to represent a desirable accuracy for providing a diagnostic test in accordance with the invention.

Table 1 below summarizes, from among all biomarkers and biomarker panels determined, the data representing an ROC AUC value of above 0.70.

The biomarker proteins presented in the table are IL1β, MMP-9, IL6, and MMP-3, as mentioned above, as well as MMP-8 and HGF.

In the table, it can be seen that maximum results, in terms of ROC AUC of 0.77 are obtained with biomarker panel consisting of three markers: IL1β, MMP-9, and IL6. A result close to this, at 0.76, is obtained with panels of four biomarkers, viz. IL1β, MMP-9, IL6, and either MMP-3 or MMP-8. Results at 0.74 and 0.75 are obtained for five marker panels. However, also the three marker panel IL1β, MMP-9, MMP-3 provides an ROC AUC of 0.74. For the purpose of providing a simple, yet adequately reliable test useful in the present invention, it is desired to apply as few biomarkers as possible. It can thereby be seen that the biomarker panels of 1 marker and 2 markers provide ROC AUC values of below, or well below, 0.7, i.e., substantially closer to the aforementioned value of a worthless test (0.5) than to a perfect test (1).

It has now been found that the optimal results, of having a ROC AUC above 0.70 applying as few markers as possible, is contingent on the selection of a biomarker panel consisting of IL1β, MMP-9, and either IL6 or MMP-3, or with further markers added as shown. Clearly, with the results being similar, the 3-marker panels IL1β, MMP-9, and either IL6 or MMP-3, are preferred over the 4-marker panels.

Table 1

| IL1β | IL6 | MMP-3 | MMP-8 | MMP-9 | HGF | ROC AUC |
|---|---|---|---|---|---|---|
| *1 marker* | | | | | | |
| X | | | | | | 0.63 |
| | | | | | X | 0.63 |
| *2 markers* | | | | | | |
| X | X | | | | | 0.64 |
| X | | X | | | | 0.64 |
| X | | | | X | | 0.69 |
| | | | | X | X | 0.66 |
| *3 markers* | | | | | | |
| X | X | | X | | | 0.66 |
| X | X | | | X | | 0.77 |
| X | | X | | X | | 0.74 |
| X | | X | | | X | 0.66 |
| X | | | X | X | | 0.66 |
| X | | | | X | X | 0.67 |
| | X | | | X | X | 0.67 |
| *4 markers* | | | | | | |
| X | X | X | | X | | 0.76 |
| X | X | | X | X | | 0.76 |
| X | X | | | X | X | 0.73 |
| X | | X | X | X | | 0.72 |
| *5 markers* | | | | | | |
| X | X | X | X | X | | 0.75 |
| X | X | X | | X | X | 0.72 |
| X | X | | X | X | X | 0.74 |
| *6 markers* | | | | | | |
| X | X | X | X | X | X | 0.72 |

While the invention has been illustrated and described in detail in the drawings and foregoing description, such illustration and description are to be considered illustrative or exemplary and not restrictive; the invention is not limited to the disclosed embodiments. For example, it is possible to present binding agents for different biomarkers in different units. Or, conveniently, a kit of the invention can comprise a fixed set of binding agents for the protein biomarkers that are used in all embodiments, i.e., MMP-9 and IL1β, and flexible modules comprising a binding agent for either of the further biomarkers, i.e., MMP-8 or MMP-3.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose an *in vitro* method for assessing whether a human patient suffering from periodontitis has mild periodontitis or advanced periodontitis. The method is based on the insight to determine a selection of three biomarker proteins. Accordingly, in a sample of saliva a patient suffering from periodontitis, the concentrations are measured of the proteins Interleukin-1β (IL1β), Matrix metalloproteinase 9 (MMP-9) and at least one of the proteins: Interleukin-6 (IL6), and Matrix metalloproteinase 3 (MMP-3). Based on the concentrations as measured, a value is determined reflecting the joint concentrations for said proteins. This value is compared with a threshold value reflecting in the same manner the joint concentrations associated with advanced periodontitis. The comparison allows assessing whether the testing value is indicative of the presence of advanced periodontitis or of mild periodontitis in said patient. Thereby, typically, a testing value reflecting a joint concentration below the joint concentration reflected by the threshold value is indicative for mild periodontitis in said patient, and a testing value reflecting a joint concentration at or above the joint concentration reflected by the threshold value, is indicative for advanced periodontitis in said patient.

**Claims**

1. An *in vitro* method for assessing whether a human patient, known to have periodontitis, has mild periodontitis or advanced periodontitis, wherein the method comprises detecting, in a sample of saliva from said patient, the concentrations of the proteins: Interleukin-1β (IL1β), Matrix metalloproteinase 9 (MMP-9), and at least one of the proteins Interleukin-6 (IL6) and Matrix metalloproteinase 3 (MMP-3); determining a testing value reflecting the joint concentrations determined for said proteins; comparing said value with a threshold value reflecting in the same manner the joint concentrations associated with advanced periodontitis, so as to assess whether the indicative for mild periodontitis or for advanced periodontitis in said patient.

2. A method according to claim 1, wherein the threshold value is based on the concentrations determined for the proteins in a reference sample associated with the presence of advanced periodontitis.

3. A method according to claim 1, wherein the threshold value is based on the concentrations of the proteins in a set of samples, including subjects that have mild or moderate periodontitis and subjects having advanced periodontitis.

4. A method according to any one of the preceding claims, wherein the proteins consist of IL1β, MMP-9, IL6, and one of the proteins MMP-3 and MMP-8.

5. A method according to any one of the claims 1 to 3, wherein the proteins consist of IL1β, MMP-9, and IL6.

6. A method according to any one of the claims 1 to 3, wherein the proteins consist of IL1β, MMP-9, and MMP-3.

7. A method according to any one of the preceding claims, wherein the concentration values determined are arithmetically processed into a number between 0 and 1.

8. The use of the proteins Interleukin-1β (IL1β), Matrix metalloproteinase 9 (MMP-9), and at least one of the proteins Interleukin-6 (IL6) and Matrix metalloproteinase 3 (MMP-3), as biomarkers in a saliva sample of a human patient suffering from periodontitis, for assessing whether the patient has mild periodontitis or advanced periodontitis.

9. A system for classifying the type of periodontitis in a human patient suffering from said periodontitis, the system comprising:

    - a container for receiving a saliva sample of a subject therein, the container provided with detection means which are able and adapted to detect in the oral sample an expression of a set of biomarkers, the set comprising IL1β, MMP-9, and at least one of IL6 and MMP-3;
    - a data connection to an interface, particularly a graphical user interface, capable of presenting information, said interface being either a part of the system or a remote interface;
    - a processor able and adapted to determine from the detected expression of the set of biomarkers in an oral sample of the patient a diagnosis result comprising an indication of the patient having mild periodontitis or advanced periodontitis.

10. A system according to claim 9, wherein the processor is enabled to function by means of an internet-based application.

11. A kit for determining the concentration of at least three biomarkers for periodontitis in saliva, said kit comprising three binding agents, each binding agent directed to a different biomarker, wherein a first binding agent is capable of binding IL1β, a second binding agent is capable of binding MMP-9, and a third binding agent is capable of binding one of IL6 and MMP-3.

**12.** A kit according to claim 11, wherein said binding agents are contained on a solid support, such as a chip, a microtiter plate a bead or a resin.

**13.** A kit according to claim 11 or 12, wherein the binding agents consist of binding agents for IL1$\beta$, MMP-9, and IL6.

**14.** A kit according to claim 11 or 12, wherein the binding agents consist of binding agents for IL1$\beta$, MMP-9, and MMP-3.

**15.** An *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins: IL1$\beta$, MMP-9, and at least one of IL6 and MMP-3, and comparing the concentrations, whereby a difference in any one, two, or all three of the concentrations, reflects a change in status.

Fig. 1

Europäisches
Patentamt
European
Patent Office
Office européen
des brevets

## EUROPEAN SEARCH REPORT

Application Number

EP 17 17 2735

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/196941 A1 (BRAUN THOMAS [US] ET AL) 5 August 2010 (2010-08-05) * par. 0025; examples 1, 3 and 4; tables 4 and 10, claim 12 * | 1-5,8,9, 11-13,15 | INV. G01N33/68 |
| X | CHRISTOPH A. RAMSEIER ET AL: "Identification of Pathogen and Host-Response Markers Correlated With Periodontal Disease", JOURNAL OF PERIODONTOLOGY., vol. 80, no. 3, 1 March 2009 (2009-03-01), pages 436-446, XP055400653, US ISSN: 0022-3492, DOI: 10.1902/jop.2009.080480 * abstract, fig. 1, tables 3-5 * | 1-3,8, 11,12 | |
| X | THIAGO MORELLI ET AL: "Salivary Biomarkers in a Biofilm Overgrowth Model", JOURNAL OF PERIODONTOLOGY., vol. 85, no. 12, 1 December 2014 (2014-12-01), pages 1770-1778, XP055427552, US ISSN: 0022-3492, DOI: 10.1902/jop.2014.140180 * p. 1771-1773 Table 3 p. 1777 * | 1-4, 6-10,12, 14,15 | |
| X | SUHEE KIM ET AL: "Differential Matrix Metalloprotease (MMP) Expression Profiles Found in Aged Gingiva", PLOS ONE, vol. 11, no. 7, 1 August 2016 (2016-08-01) , page e0158777, XP055427557, DOI: 10.1371/journal.pone.0158777 * abstract and Fig. 4b * | 1-4, 6-12,14, 15 | |

TECHNICAL FIELDS SEARCHED (IPC)

G01N

-/--

The present search report has been drawn up for all claims

| Place of search | Date of completion of the search | Examiner |
|---|---|---|
| Munich | 27 November 2017 | Schalich, Juliane |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt

European Patent Office

Office européen des brevets

# EUROPEAN SEARCH REPORT

Application Number

EP 17 17 2735

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MAÉLSON KLEVER DA SILVA ET AL: "Genetic Factors and the Risk of Periodontitis Development: Findings from a Systematic Review Composed of 13 Studies of Meta-Analysis with 71,531 Participants", INTERNATIONAL JOURNAL OF DENTISTRY, vol. 2017, 1 January 2017 (2017-01-01), pages 1-9, XP055427554, United States ISSN: 1687-8728, DOI: 10.1155/2017/1914073 * the whole document * ----- | 1-4, 6-12,14, 15 | |

TECHNICAL FIELDS SEARCHED (IPC)

The present search report has been drawn up for all claims

| Place of search | Date of completion of the search | Examiner |
|---|---|---|
| Munich | 27 November 2017 | Schalich, Juliane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

1. claims: 5, 13(completely); 1-4, 7-12, 15(partially)

    distinguishing between mild and advanced periodontitis by using the combination of at the following 3 markers: Interleukin-1 ? (IL 1 ?), Matrix metalloproteinase 9 (MMP-9), Interleukin-6 (IL6)
    ---

2. claims: 6, 14(completely); 1-4, 7-12, 15(partially)

    distinguishing between mild and advanced periodontitis by using the combination of at the following 3 markers: Interleukin-1 ? (IL 1 ?), Matrix metalloproteinase 9 (MMP-9), Matrix metalloproteinase 3 (MMP-3).
    ---

## ANNEX TO THE EUROPEAN SEARCH REPORT
## ON EUROPEAN PATENT APPLICATION NO.

EP 17 17 2735

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2017

| Patent document cited in search report | Publication date | Patent family member(s) | Publication date |
|---|---|---|---|
| US 2010196941 A1 | 05-08-2010 | US 2010196941 A1<br>WO 2009018342 A1 | 05-08-2010<br>05-02-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MORGAN RG.** *Brit Dent J,* 2001, vol. 191, 436-41